# DEMANDE DE BREVET EUROPEEN

(11) **EP 3 831 241 A1**
(43) Date de publication de la demande: **09.06.2021**
(21) Numéro de dépôt: 20210362.8
(22) Date de dépôt: 27.11.2020
(51) Int. Cl.: A45D 26/00

(54) **APPAREIL DE CHAUFFAGE D'UNE BANDE EPILATOIRE, KIT D'EPILATION ET PROCEDE DE CHAUFFAGE ASSOCIES**

(30) Priorité: 04.12.2019 FR 1913755
(71) Demandeur: SEB S.A., 69130 Ecully (FR)
(72) Inventeur: DELHOM, Jérémy, 69004 LYON (FR)
(74) Mandataire: SEB Développement

(57) **Abrégé**

- L'invention concerne un appareil de chauffage (1) d'une bande épilatoire (2) comprenant au moins une couche de substance épilatoire (5), caractérisé en ce qu'il comprend:
o un premier rouleau d'entrainement (9) conçu pour être mis en rotation de façon à déplacer ladite bande épilatoire, et
o un organe de maintien (12) pour maintenir en contact une première zone d'entrainement (13) de ladite bande épilatoire avec une première portion d'entrainement (14) dudit premier rouleau,
le premier rouleau et/ou l'organe de maintien comprenant une portion évidée (16) pour accueillir ladite couche (5), l'appareil comprenant un moyen de chauffage (18) portant ladite substance épilatoire à température d'utilisation lorsque ladite couche (5) est dans ladite portion évidée.

- L'invention est particulièrement adaptée pour chauffer efficacement et en toute sécurité une bande épilatoire.

## Description

La présente invention se rapporte au domaine technique général de l'élimination des poils superflus, et plus précisément à un appareil pour faciliter l'épilation humaine, en particulier à la cire.

L'invention concerne ainsi un appareil de chauffage d'au moins une bande épilatoire, ladite bande épilatoire comprenant au moins un premier substrat et une couche de substance épilatoire, par exemple une cire, préenduite sur ledit premier substrat.

La mise en oeuvre de techniques d'épilation par application de bandes de cire chaude est connue. La cire, contenue dans un pot, est chauffée au four à micro-ondes afin d'être ramollie, puis appliquée sous forme de couches sur une zone à épiler d'un individu jusqu'à durcissement, les couches formant alors des bandes se solidarisant avec les poils, les bandes étant ensuite décollées de la peau pour en arracher les poils.

Néanmoins, la cire chaude en pot est très salissante et nécessite un accessoire spécifique d'application, tel qu'une spatule, qu'il faut parfois nettoyer après usage, et qui peut être égaré. De surcroît, il est particulièrement difficile de maîtriser la chauffe d'un pot de cire au four à micro-ondes tant en termes d'uniformité que de niveau de température, ce qui entraîne un risque de surchauffe globale ou localisée de la cire en pot, pouvant conduire à un inconfort d'application, voire à abîmer la peau. De plus, la cire chaude en pot ne convient pas pour un usage nomade, par exemple lorsqu'un individu voulant s'épiler est en déplacement et ne dispose pas d'un four à micro-ondes. En outre, le risque de gaspiller une grande quantité de cire chaude est particulièrement élevé lorsque l'utilisateur applique trop de cire chaude ramollie sur une zone à épiler, ce qui est relativement fréquent, la dose de cire chaude à appliquer étant difficile à évaluer avec précision et peu reproductible d'une application à une autre. Il est de surcroît nécessaire de réchauffer tout le pot de cire pour pouvoir l'utiliser, même lorsqu'il n'y a qu'une faible surface à épiler, ce qui rend le processus d'épilation long, fastidieux et de moins en moins efficace, puisque la cire non utilisée se dégrade à chaque chauffage.

Ainsi, d'autres alternatives d'épilation plus douces et plus pratiques ont été développées. Parmi ces alternatives, une technique courante est l'application sur une zone à épiler de bandes épilatoires prédécoupées dites « *bandes de cire froides* », chacune formée de deux feuilles de substrat entre lesquelles est emprisonnée une couche de cire. L'utilisation de ces bandes est extrêmement simple et ne demande aucun matériel particulier : il suffit pour l'utilisateur voulant s'épiler de réchauffer la bande entre ses mains ou contre une autre partie de son corps, puis de séparer les deux feuilles de substrats. Ceci libère la couche de cire et la sépare donc en deux épaisseurs de cire, recouvrant chacune un côté respectif d'une feuille de substrat respective. Il suffit ensuite d'appliquer l'épaisseur de cire contre la zone à épiler, d'attendre quelques secondes que la cire refroidisse et se solidarise aux poils, puis d'arracher de la peau l'ensemble comprenant la feuille de substrat, l'épaisseur de cire supportée par la feuille, et les poils de la zone à épiler, lesquels sont donc emportés par l'épaisseur de cire.

Ces techniques et systèmes connus d'épilation avec des bandes d'épilation, bien que donnant globalement satisfaction, n'en présentent pas moins certains désavantages.

En effet, les bandes de cire froide présentent une efficacité assez faible et leur utilisation peut être relativement longue, puisqu'il est nécessaire de les chauffer une à une contre le corps puis de les appliquer sur les zones à épiler. Le réchauffage par la chaleur corporelle s'avère en réalité non seulement insuffisant, mais également peu durable, irrégulier et difficilement répétable, chaque bande étant chauffée à des températures différentes et à différents endroits. De plus, les bandes de cire froide se refroidissent extrêmement vite une fois qu'elles ne sont plus en contact avec le corps, perdant ainsi de leur efficacité avant même leur application sur la zone à épiler.

Les objets assignés à la présente invention visent en conséquence à remédier aux différents inconvénients énumérés précédemment et à proposer un nouvel appareil de chauffage d'au moins une bande épilatoire qui, tout en étant de construction particulièrement fiable et compacte, présente une mise en oeuvre particulièrement facile.

Un autre objet de l'invention vise à proposer un nouvel appareil de chauffage dont la construction lui permet d'être particulièrement résistant aux dégradations liées au chauffage et à la mise en oeuvre de bandes épilatoires.

Un autre objet de l'invention vise à proposer un nouvel appareil de chauffage permettant de chauffer une bande épilatoire de manière à maximiser son efficacité.

Un autre objet de l'invention vise à proposer un nouvel appareil de chauffage qui permet de rendre prêt à l'emploi un nombre important de bandes épilatoires en une durée réduite.

Un autre objet de l'invention vise à proposer un nouvel appareil de chauffage qui permet à l'utilisateur de disposer d'un stock de bandes épilatoires prêtes à l'emploi.

Un autre objet de l'invention vise à proposer un nouvel appareil de chauffage présentant un excellent niveau de sécurité d'utilisation.

Un autre objet de l'invention vise à proposer un nouvel appareil de chauffage qui, tout en étant de construction robuste, présente une grande modularité lui permettant notamment de s'adapter à différents types de bandes épilatoires à chauffer.

Un autre objet de l'invention vise à proposer un nouvel appareil de chauffage de conception simple, et dont les coûts de fabrication sont maîtrisés.

Un autre objet de l'invention vise à proposer un nouvel appareil de chauffage permettant à l'utilisateur de disposer rapidement de bandes épilatoires convenablement chauffées, et notamment chauffées de manière régulière, reproductible et contrôlée sur leur surface.

Un autre objet de l'invention vise à proposer un nouvel appareil de chauffage de construction légère.

Un autre objet de l'invention vise à proposer un nouvel appareil de chauffage qui convient à une utilisation aussi bien nomade que sédentaire.

Un autre objet de l'invention vise à proposer un nouvel appareil de chauffage d'au moins une bande épilatoire dont l'utilisation est particulièrement simple et intuitive.

Un autre objet de l'invention vise à proposer un nouvel appareil de chauffage peu salissant et se salissant peu même après un grand nombre d'utilisations.

Un autre objet de l'invention vise à proposer un nouvel appareil de chauffage ne demandant qu'un entretien minimal, aisé à réaliser et à coûts maitrisés.

Un autre objet de l'invention vise à proposer un nouvel appareil de chauffage particulièrement ergonomique.

Un autre objet de l'invention vise à proposer un nouveau kit d'épilation compact, modulable, fiable et pratique d'utilisation, tout en présentant une grande efficacité d'épilation et un excellent niveau de sécurité d'utilisation.

Un autre objet de l'invention vise à proposer un nouveau procédé de chauffage d'au moins une bande épilatoire qui soit particulièrement simple, efficace et intuitif, tout en présentant des conditions de sécurité optimales.

Pour atteindre ces buts, l'invention concerne un appareil de chauffage d'au moins une bande épilatoire, ladite bande épilatoire comprenant au moins un premier substrat et une couche de substance épilatoire, par exemple une cire, préenduite sur ledit premier substrat, ledit appareil de chauffage étant caractérisé en ce qu'il comprend au moins :
- un premier rouleau d'entrainement conçu pour être mis en rotation de façon à déplacer ladite bande épilatoire d'une entrée d'alimentation vers une sortie d'enlèvement de l'appareil de chauffage, et
- un organe de maintien pour maintenir en contact au moins une première zone d'entrainement de ladite bande épilatoire avec au moins une première portion d'entrainement dudit premier rouleau d'entrainement, afin de permettre l'entrainement de ladite bande épilatoire par ledit premier rouleau,
   ledit premier rouleau d'entrainement et/ou ledit organe de maintien comprenant une portion évidée destinée à accueillir ladite couche lorsque ladite bande épilatoire est entrainée par ledit premier rouleau d'entrainement, ledit appareil de chauffage comprenant en outre un moyen de chauffage prévu pour chauffer ladite portion évidée de façon à porter ladite substance épilatoire à une température d'utilisation lorsque ladite couche est reçue dans ladite portion évidée.

Les objets assignés à l'invention sont également atteints à l'aide d'un kit d'épilation comprenant un appareil de chauffage et au moins une bande épilatoire tels que décrits précédemment.

Les objets assignés à l'invention sont également atteints à l'aide d'un procédé de chauffage d'au moins une bande épilatoire, ladite bande épilatoire comprenant au moins un premier substrat et une couche de substance épilatoire, par exemple une cire, préenduite sur ledit premier substrat, le procédé de chauffage étant caractérisé en ce qu'il comprend au moins :
- une étape de mise en contact de ladite bande épilatoire avec un premier rouleau d'entrainement et un organe de maintien, l'organe de maintien permettant de maintenir en contact au moins une première zone d'entrainement de ladite bande épilatoire avec au moins une première portion d'entrainement dudit premier rouleau d'entrainement,
- une étape de mise en rotation dudit premier rouleau d'entrainement de façon à entraîner ladite bande épilatoire d'une entrée d'alimentation vers une sortie d'enlèvement de l'appareil de chauffage,
- une étape d'insertion de ladite couche dans une portion évidée du premier rouleau d'entrainement et/ou de l'organe de maintien, et
- une étape de chauffage de ladite portion évidée de façon à porter ladite substance épilatoire à une température d'utilisation lorsque ladite couche est insérée dans ladite portion évidée.

D'autres objets et avantages particuliers de l'invention apparaîtront plus en détails à la lecture de la description qui suit, ainsi qu'à l'aide des dessins annexés, fournis à titre purement illustratif et non limitatif, parmi lesquels :
La figure 1 illustre, selon une vue schématique en perspective, un mode de réalisation d'un appareil de chauffage conforme à l'invention, ledit appareil incluant dans cet exemple particulier une enceinte abritant différents éléments mécaniques.
La figure 2 illustre, selon une vue schématique en perspective, l'appareil de chauffage de la figure 1 à partir d'un autre point de vue.
La figure 3 illustre, selon une vue schématique de dessous, l'appareil de chauffage de la figure 1.
La figure 4 illustre, selon une vue schématique en perspective, l'appareil de la figure 1 dont une moitié a été coupée selon le plan A illustré aux figures 1 et 3 afin de rendre visible l'intérieur de l'appareil.
La figure 5 illustre, selon une vue schématique en perspective de l'appareil de la figure 4 mais depuis un autre point de vue.
La figure 6 illustre, selon une vue schématique en coupe, l'intérieur de l'appareil de chauffage de la figure 1, c'est-à-dire l'appareil de chauffage de la figure 1 dépourvu d'enceinte.
La figure 7 illustre, selon une vue schématique en perspective, l'appareil de chauffage de la figure 6 sans son moteur d'entrainement.
La figure 8 illustre, selon une vue en coupe selon le plan A, l'appareil de chauffage de la figure 1.
La figure 9 illustre, selon une vue schématique en perspective, l'appareil de chauffage de la figure 6 sans son système d'entrainement.
La figure 10 illustre, selon une vue schématique en perspective, une partie de l'appareil de chauffage de la figure 1, et notamment l'appareil de chauffage de la figure 6 dont certains éléments intérieurs auraient été retirés.
La figure 11 illustre, selon une vue schématique en perspective, une partie de l'appareil de chauffage de la figure 10.
La figure 12 illustre, selon une vue schématique du dessus, un exemple de bande épilatoire conforme à un kit d'épilation de l'invention et utilisable par l'appareil de chauffage des figures précédentes, avec une feuille de protection en train d'être enlevée pour libérer une couche de substance épilatoire supportée par une feuille de substrat.
La figure 13 illustre, selon une vue schématique en coupe, une coupe selon le plan B de la bande épilatoire de la figure 12, avant séparation.
La figure 14 illustre, selon une vue schématique en perspective, un autre exemple de bande épilatoire, conforme à un kit d'épilation de l'invention et ayant donc été chauffée par un appareil de chauffage de l'invention, et dont deux feuilles de substrats chacune enduite de substance épilatoire sont séparées l'une de l'autre pour libérer la substance épilatoire en deux couches distinctes.

L'invention concerne, selon un premier aspect, un appareil de chauffage 1 d'au moins une bande épilatoire 2. Bien évidemment, l'appareil de chauffage 1 de l'invention est avantageusement apte à chauffer plusieurs bandes épilatoires 2, par exemple l'une après l'autre ou alternativement plusieurs en parallèle. L'appareil de chauffage 1 peut être utilisé aussi bien par des particuliers, ayant besoin de s'épiler eux-mêmes par exemple, que par des professionnel(le)s de l'épilation, comme des esthéticiennes.

L'invention concerne, selon un deuxième aspect, un kit d'épilation comprenant un appareil de chauffage 1 et au moins une bande épilatoire 2, tels que décrits ci-avant et ci-après.

Chaque bande épilatoire 2 est de préférence, dans les modes de réalisation préférés de l'invention, formée par une « *bande de cire froide* » dont l'utilisation dans l'épilation est connue. Ladite bande épilatoire 2 peut ainsi soit être achetée dans le commerce, soit être réalisée spécifiquement pour être chauffée par l'appareil de chauffage 1 de l'invention, et dans les deux cas faire partie de l'invention et notamment du kit d'épilation. Ladite bande épilatoire 2 se présente avantageusement sous la forme d'une pièce plate, préférentiellement sensiblement souple, formée d'une ou plusieurs épaisseurs distinctes. En particulier, ladite bande épilatoire 2 est discrète, présentant une épaisseur assez réduite, par exemple inférieure à 1 cm, avantageusement inférieure à 0,5 cm, de manière plus avantageuse inférieure à 2 mm, et de façon encore plus préférentielle inférieure à 0,5 mm, et une largeur et une longueur de quelques centimètres, par exemple chacune comprise entre 3 et 40 cm, de préférence entre 4 et 25 cm. Ladite bande épilatoire 2 est par exemple prédécoupée en plusieurs pièces discrètes distinctes à partie d'une bande brute, ladite bande brute pouvant optionnellement former un rouleau. De manière préférentielle, le kit d'épilation comprend donc une pluralité de bandes épilatoires 2 distinctes, de préférence prédécoupées, ou alternativement formées indépendamment les unes des autres. La description qui précède, comme celle qui suit, concernant l'appareil de chauffage 1 s'applique donc également au kit d'épilation selon l'invention, et inversement, la description qui suit concernant le kit d'épilation s'applique avantageusement également à l'appareil de chauffage 1 selon l'invention. De façon avantageuse, ladite bande épilatoire 2 ne constitue pas en tant que telle un rouleau, bien que cette possibilité ne soit pas totalement exclue, la bande épilatoire 2 pouvant éventuellement constituer un rouleau dans un mode de réalisation particulier de l'invention.

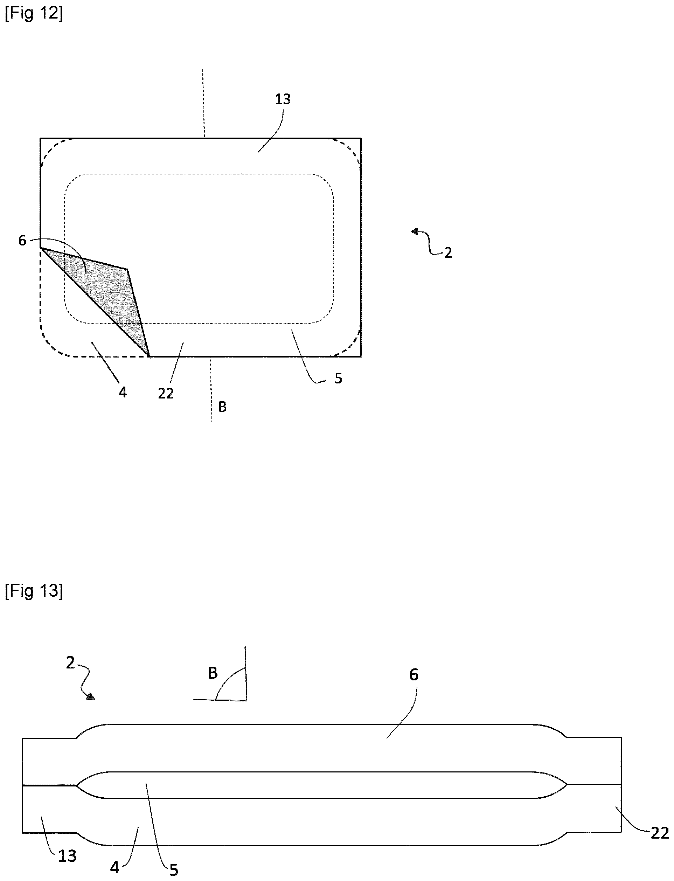

Selon l'invention, ladite bande épilatoire 2 comprend au moins un premier substrat 4 et une couche de substance épilatoire 5, par exemple une cire, préenduite sur ledit premier substrat 5, comme illustré aux figures 12 à 14.

La substance épilatoire est donc préférentiellement une cire utilisable dans l'épilation, et en particulier dans l'épilation par arrachage mécanique des poils. L'appareil de chauffage 1 permet avantageusement de chauffer la couche de substance épilatoire 5 de manière à augmenter son efficacité d'arrachage des poils, par exemple en ramollissant suffisamment ladite couche 5 pour que la substance épilatoire emprisonne les poils ou colle avec ces derniers avant de refroidir et s'affermir pour permettre leur arrachement. De manière avantageuse, la substance épilatoire est une matière thermoplastique, et donc se ramollit, devient sensiblement fluide ou moins visqueuse, lorsqu'elle est chauffée à une certaine température de transition (par exemple une température d'utilisation comme cela sera décrit plus tard) ou au-dessus, mais reste sensiblement dure ou extrêmement visqueuse en-dessous de cette température de transition. Les matières, telles que les cires et substances thermoplastiques, pouvant constituer la substance épilatoire, sont pour la plupart couramment utilisées, notamment dans des « *bandes de cire froide* », et ne seront pas décrites plus avant. Ladite température de transition peut être comprise entre 25 et 60°C, plus préférentiellement entre 30 et 50 °C, par exemple environ 40°C (+/-3°C). Alternativement, ladite substance épilatoire est formée par un composé dépilatoire conçu pour détruire chimiquement (par désintégration et/ou dissolution par exemple) les poils, pour autant que l'action dépilatoire de ce composé doive être activée par chauffage dudit composé à une température d'utilisation (qui peut être celle décrite ci-après), ce chauffage pouvant bien évidemment être effectué par l'appareil de chauffage 1 de l'invention. Lorsque la substance épilatoire est formée par ce composé, elle est donc de type *« dépilatoire* », c'est-à-dire qu'elle permet une épilation par destruction chimique des poils et non par arrachage mécanique, comme avec la cire mentionnée précédemment par exemple.

Le premier substrat 4 est de préférence formé par une première feuille souple 4, et est avantageusement réalisé en un matériau qui ne fond pas à la même température que la substance épilatoire mais à une température supérieure voire pas du tout, le premier substrat 4 n'ayant préférentiellement pas le comportement d'une cire. En particulier, le premier substrat 4 reste solide lorsque la couche de substance épilatoire 5 commence à fondre et/ou se ramollir sous l'action de l'appareil de chauffage 1, par exemple vers 40°C. La couche de substance épilatoire 5 est donc préférentiellement préenduite sur une face dudit premier substrat 4.

De manière avantageuse, ladite bande épilatoire 2 comprend en outre une feuille protectrice 6, laquelle recouvre le premier substrat 4 du côté de ladite couche de substance épilatoire 5. La feuille protectrice 6 recouvre donc avantageusement aussi ladite couche de substance 5, notamment pour la protéger, éviter sa dégradation, et l'empêcher de coller à des éléments de son environnement avant utilisation. Ladite feuille protectrice 6 peut éventuellement être formée par un deuxième substrat 6 de même nature ou d'une nature différente de celle du premier substrat 4, et est préférentiellement souple. Ladite feuille protectrice 6 ne présente avantageusement pas un comportement de cire et notamment ne fond pas / ne se ramollit pas comme la couche de substance épilatoire 5 lorsque celle-ci est chauffée par l'appareil de chauffage 1.

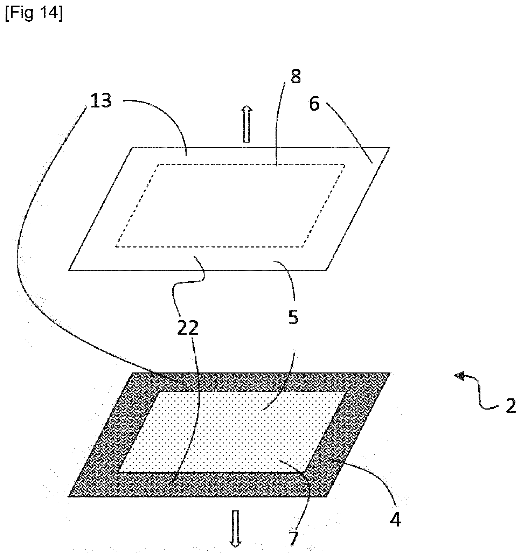

De préférence, avant utilisation dans le cadre d'une opération d'épilation, mais pendant le chauffage par l'appareil de chauffage 1, ladite couche de substance épilatoire 5 est emprisonnée entre ledit premier substrat 4 et ladite feuille protectrice 6, comme cela est illustré aux figures 12 à 14. La figure 12 illustre une bande d'épilation 2 chauffée prête à l'emploi, dans laquelle la feuille protectrice 6 est en train d'être séparée du premier substrat 4 (sous forme d'une première feuille souple). Optionnellement, notamment lorsque ladite feuille protectrice 6 est formée par un deuxième substrat 6, et pendant une opération d'épilation, après le chauffage par l'appareil de chauffage 1, la couche 5, précédemment emprisonnée entre lesdits premier et deuxième substrats 4, 6, se sépare en deux sous-couches de substance épilatoire 7, 8 recouvrant chacune une portion respective desdits premier et deuxième substrats 4, 6, comme cela est illustré à la figure 14. Une telle configuration permet d'obtenir deux substrats 4, 6 chacun enduit de substance épilatoire.

La couche de substance épilatoire 5 n'occupe avantageusement qu'une portion de la face du premier substrat 4, de façon que les bords du premier substrat 4 soient collés aux bords de la feuille protectrice 6, emprisonnant ainsi, avant utilisation de la bande épilatoire 2, la couche de substance épilatoire 5 entre ledit premier substrat 4 et ladite feuille protectrice 6, comme l'illustrent les figures 12 et 13. Pour réaliser l'opération d'épilation, il est donc de préférence nécessaire, après chauffage de la bande épilatoire 2 par l'appareil de chauffage 1, de séparer la feuille protectrice 6 du premier substrat 4 notamment en décollant les bords correspondants, comme illustré à la figure 12. Ledit premier substrat 4 et/ou ladite feuille protectrice présente(nt) (chacun) par exemple une forme globalement rectangulaire respective, comme illustré aux figures 12 et 14, ou bien encore une forme elliptique ou toute autre forme appropriée pour l'épilation à l'aide de bande épilatoire discrète. Préférentiellement, ladite bande épilatoire 2 présente une forme sensiblement allongée, mais elle peut aussi alternativement être carrée voire ronde. Optionnellement, ledit premier substrat 4 présente un ou plusieurs coins arrondis, pour le décoller plus facilement de la feuille protectrice 6 qui n'en présente pas, comme illustré à la figure 12, ou inversement, la feuille protectrice 6 présente au moins un coin arrondi tandis que le premier substrat 4 n'en présente pas.

Selon l'invention, l'appareil de chauffage 1 comprend au moins :
- un premier rouleau d'entrainement 9 conçu pour être mis en rotation de façon à déplacer ladite bande épilatoire 2 d'une entrée d'alimentation 10 vers une sortie d'enlèvement 11 de l'appareil de chauffage 1, et
- un organe de maintien 12 pour maintenir en contact au moins une première zone d'entrainement 13 de ladite bande épilatoire 2 avec au moins une première portion d'entrainement 14 dudit premier rouleau d'entrainement 9, afin de permettre l'entrainement de ladite bande épilatoire 2 par ledit premier rouleau 9.

Ainsi, de façon préférentielle, l'entrainement de la bande épilatoire 2 se fait par et au sein de l'appareil de chauffage 1, à l'aide au moins de la rotation du premier rouleau d'entrainement 9, lequel reste donc en contact avec ladite bande épilatoire 2 pendant son déplacement grâce à l'organe de maintien 12, lequel plaque au moins ladite première zone d'entrainement 13 de la bande épilatoire 2 contre ladite première portion d'entrainement 14 dudit premier rouleau d'entrainement 9. Bien entendu, ladite première portion d'entrainement 14, faisant partie du premier rouleau 9, est avantageusement aussi mise en rotation lorsque ledit premier rouleau 9 l'est, et tourne autour du même axe de rotation. Ledit premier rouleau 9 est de manière avantageuse mis en rotation selon un axe de rotation R₁. L'appareil de chauffage 1 est de préférence conçu pour réaliser le déplacement de ladite bande épilatoire 2 selon une direction de d'entrainement E, laquelle est selon un mode de réalisation préféré, sensiblement perpendiculaire audit premier axe de rotation R₁. En d'autres termes, le déplacement de ladite bande épilatoire 2 se fait, selon ce mode de réalisation préféré, sensiblement perpendiculairement au moins au premier rouleau 9, bien qu'alternativement ce déplacement pourrait être oblique.

De préférence, ladite première zone d'entrainement 13 de ladite bande épilatoire 2 est sensiblement dépourvue de substance épilatoire. Ladite première zone d'entrainement 13 est de préférence formée par un premier bord de ladite bande épilatoire 2. Ladite première zone d'entrainement 13 correspond par exemple à une zone où sont collées directement l'un à l'autre un bord du premier substrat 4 et un bord de la feuille protectrice 6, comme illustré aux figures 12 à 14 et particulièrement visible à la figure 13. Ces figures, et en particulier dans la figure 13, sont schématiques, et les dimensions représentées ne correspondent pas forcément aux dimensions réelles, même relatives, des différents éléments de la bande épilatoire 2 (premier substrat 4, couche 5, feuille protectrice 6, etc.).

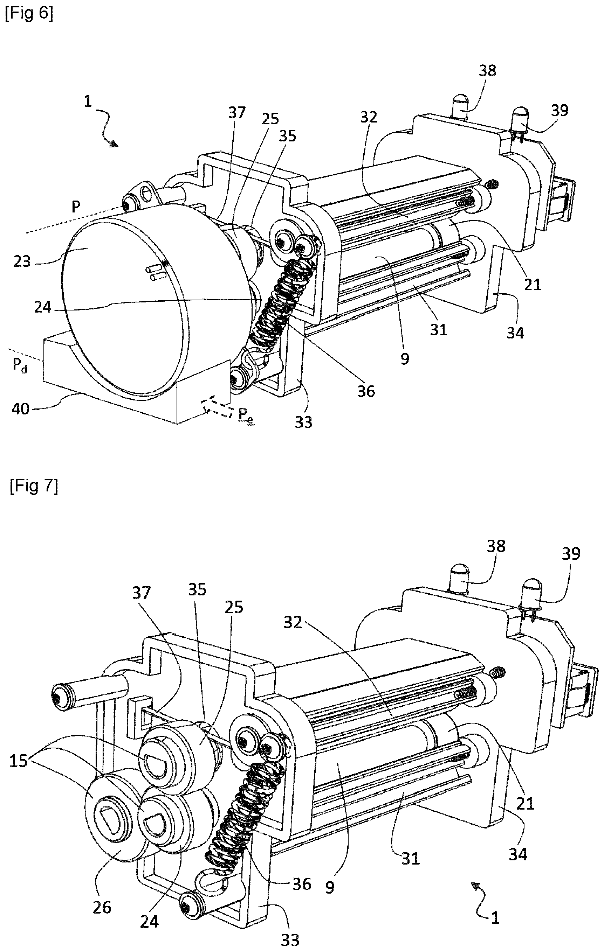

Selon un mode de réalisation préférentiel de l'invention illustré aux figures, ledit organe de maintien 12 est formé par un deuxième rouleau d'entrainement 12. Préférentiellement, ledit appareil de chauffage 1 comprend en outre un système d'entrainement 15 desdits premier et deuxième rouleaux 9, 12 en mouvement contrarotatif l'un par rapport à l'autre pour entrainer le déplacement de ladite bande épilatoire 2. De préférence, le deuxième rouleau 9 présente un deuxième axe de rotation R₂, lesdits premier et deuxième axes de rotation R₁, R₂ étant de préférence parallèles, et/ou en tous cas avantageusement distincts l'un de l'autre, c'est-à-dire non confondus (et de préférence non sécants). Le système d'entrainement 15 peut comprendre, dans le mode de réalisation illustré à la figure 7 notamment, un premier et un deuxième pignon d'entrainement 24, 25 rattachés respectivement auxdits premier et deuxième rouleaux 9, 12, de préférence dans leur prolongement respectif. Par ailleurs, ledit appareil de chauffage 1 comprend en outre avantageusement au moins un moteur d'entrainement 23 pour entrainer en rotation au moins ledit premier rouleau 9, et de préférence également ledit deuxième rouleau 12, ledit moteur d'entrainement 23 comprenant de préférence un arbre rotatif de sortie. Par exemple, le système d'entrainement 15 comprend une chaîne cinématique, incluant notamment lesdits premier et deuxième pignons d'entrainement 24, 25 et/ou un élément rotatif récepteur 26 de l'arbre rotatif de sortie, pour transformer une rotation dudit arbre rotatif de sortie dudit moteur 23 en mouvement de rotation dudit premier rouleau 9, et de préférence dudit deuxième rouleau 12 également, le mouvement de rotation dudit premier rouleau 9 et celui dudit deuxième rouleau 12 étant contrarotatifs. Pour cela, ladite chaîne cinématique peut être formée par un engrenage incluant l'élément rotatif récepteur 26 et lesdits premier et deuxième pignons d'entrainement 24, 25, associés les uns aux autres dans cet ordre (élément rotatif récepteur 26 - premier pignon 24 - deuxième pignon 25), comme illustré à la figure 7, chaque élément de l'engrenage effectuant ainsi un mouvement contrarotatif par rapport à un autre élément de l'engrenage auquel il est directement associé. Alternativement ou en combinaison, le système d'entrainement comporte une courroie de transmission. Selon une autre alternative encore, compatible avec la précédente, ledit appareil de chauffage 1 comprend deux moteurs d'entrainement distincts, chacun associé à l'un desdits premier et deuxième rouleaux 9, 12, afin de les mettre en rotation selon des mouvements contrarotatifs l'un par rapport à l'autre. Préférentiellement, lesdits premier et deuxième rouleaux 9, 12 sont agencés en regard l'un de l'autre et le long l'un de l'autre, préférentiellement parallèlement l'un à l'autre.

Selon un mode de réalisation alternatif non illustré, ledit organe de maintien 12 est formé par un deuxième rouleau libre, lequel peut présenter toutes les caractéristiques dudit deuxième rouleau d'entrainement 12 à l'exception de fait que le deuxième rouleau libre n'est pas entrainé par ledit moteur 23, mais laissé libre à rotation selon le deuxième axe de rotation R₂. Selon un autre mode de réalisation alternatif non illustré, ledit organe de maintien 12 est formé par au moins une feuille de placage pour plaquer la première zone d'entrainement 13 contre ledit premier rouleau 9, et plus précisément contre ladite première portion d'entrainement 14 dudit premier rouleau 9. Cette feuille de placage, par exemple en caoutchouc, présente à la fois une souplesse suffisante pour permettre le passage de la bande épilatoire 2 entre ledit premier rouleau 9 et la feuille de placage, et une tenue mécanique suffisante pour exercer une force de maintien ou de placage de la bande épilatoire 2 contre ledit premier rouleau 9.

Optionnellement, l'organe de maintien 12 ne comprend pas une mais plusieurs feuilles de placage. Selon encore une autre alternative, l'organe de maintien 12 comprend au moins ladite au moins une feuille de placage et ledit deuxième rouleau 12. Quel que soit le mode de réalisation, ledit premier rouleau 4, et plus précisément sa première portion d'entrainement 14, ainsi que ledit organe de maintien 12, sont préférentiellement conçus pour agir par coopération (et notamment par pincement) sur une partie dudit premier substrat 4 formée au moins par la première zone d'entrainement 14, afin de mettre en mouvement ladite bande épilatoire 2.

Selon l'invention, ledit premier rouleau d'entrainement 9 et/ou ledit organe de maintien 12 comprend une portion évidée 16 destinée à accueillir ladite couche 5 lorsque ladite bande épilatoire 2 est entrainée par ledit premier rouleau d'entrainement 9. Ainsi, l'appareil de chauffage 1 présente avantageusement ladite portion évidée 16, qui sépare de préférence au moins une partie dudit premier rouleau 9 d'une partie dudit organe de maintien 12. En particulier, ladite portion évidée 16 peut désigner un évidement, notamment au sein dudit rouleau d'entrainement 9 et/ou dudit organe de maintien 12, mais également de manière plus générale un espace libre défini au moins en partie par ledit premier rouleau d'entrainement 9 et/ou ledit organe de maintien 12, ladite portion évidée 16 pouvant désigner en outre (et en combinaison avec ce qui précède) une partie au moins dudit premier rouleau d'entrainement 9 et/ou dudit organe de maintien 12, et en particulier une partie au moins du premier rouleau d'entrainement 9 située entre les première et deuxième portions d'entrainement 14, 21 qui seront décrites plus tard.

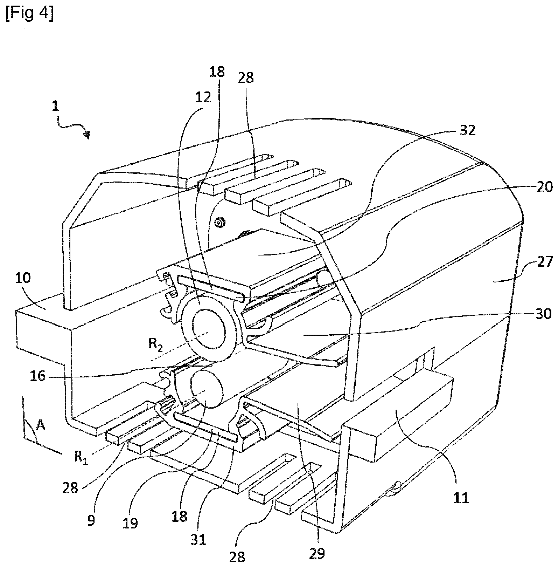

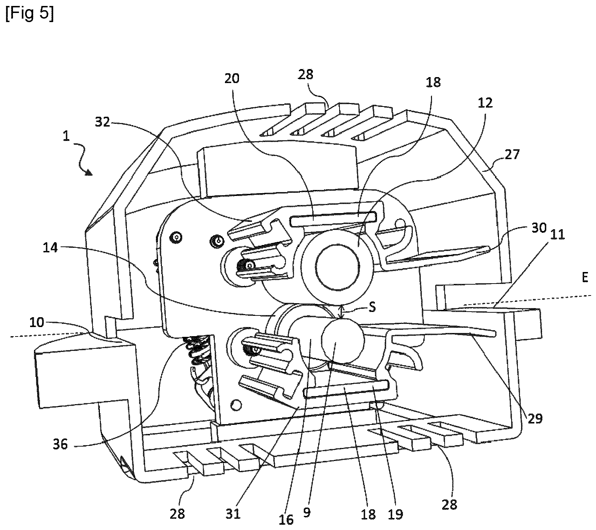

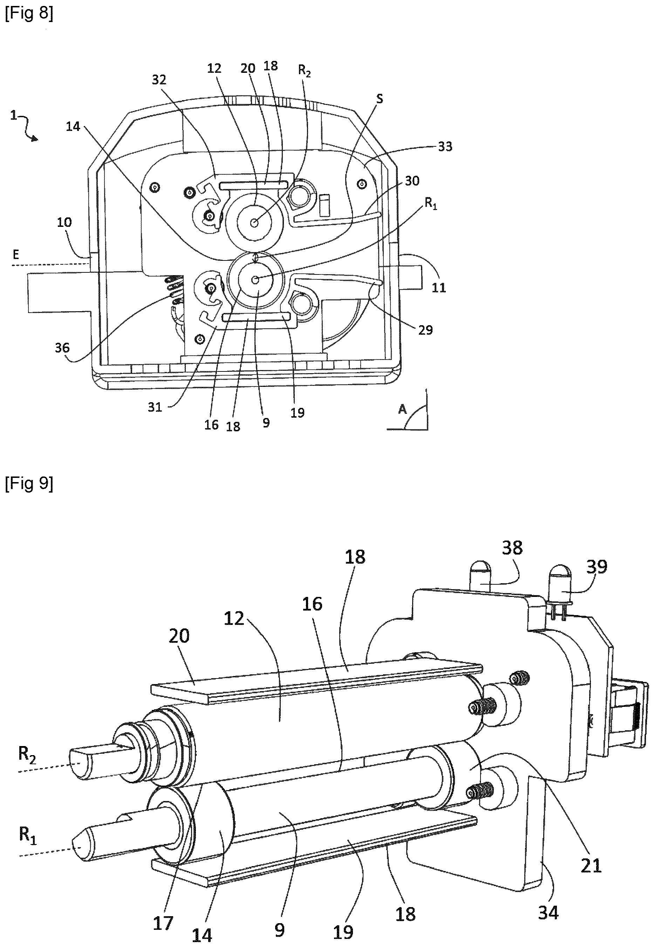

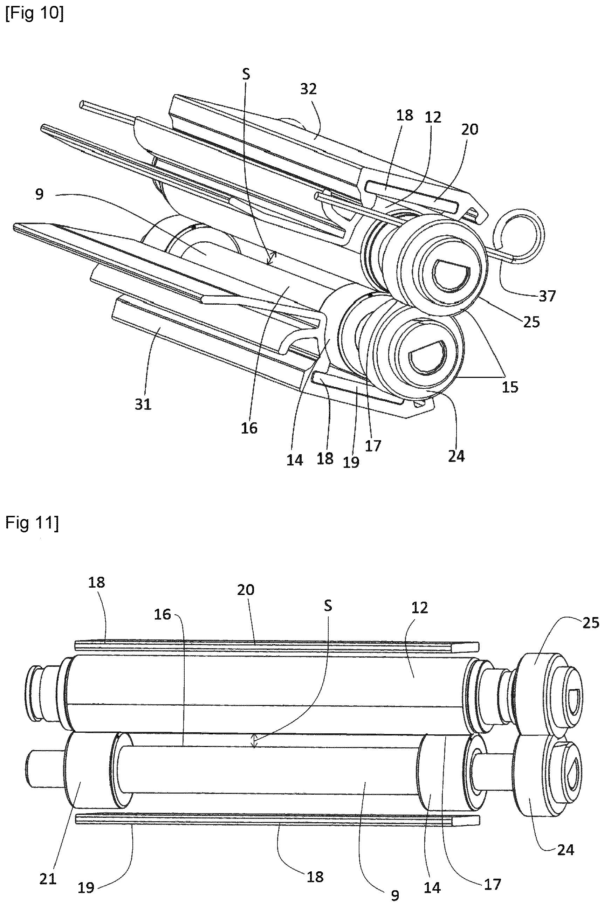

Selon un premier mode de réalisation, illustré aux figures et particulièrement visible aux figures 4, 5, et 9 à 11, ladite portion évidée 16 est prévue dans le premier rouleau 9 seulement. Selon un deuxième mode de réalisation non illustré, ladite portion évidée 16 est prévue dans l'organe de maintien 12 seulement, et plus avantageusement dans le deuxième rouleau d'entrainement 12 seulement. Selon un troisième mode de réalisation non illustré, ladite portion évidée 16 est prévue à la fois dans le premier rouleau 9 et l'organe de maintien 12, c'est-à-dire qu'une première partie de la portion évidée 16 est prévue dans le premier rouleau 9 tandis qu'une seconde partie de la portion évidée 16, complémentaire de la premier partie, est prévue dans l'organe de maintien 12 (et plus avantageusement dans ledit deuxième rouleau 12). Dans le premier mode de réalisation mentionné précédemment, le deuxième rouleau 12 peut présenter une forme générale sensiblement cylindrique à base circulaire, tandis que dans le deuxième mode, le premier rouleau 9 peut présenter une forme sensiblement cylindrique à base circulaire.

Cette configuration permet notamment à l'appareil de chauffage 1 d'entrainer la bande épilatoire 2 par le premier rouleau 9 de façon que, pendant l'entrainement de la bande épilatoire 2, la couche de substance épilatoire 5 vienne se loger, au moins en partie, au sein la portion évidée 16. Ceci permet à ladite couche de substance épilatoire 5 de ne pas être dégradée, et notamment écrasée entre lesdits premier rouleau 9 et organe de maintien 12 (et plus avantageusement entre lesdits premier et deuxième rouleaux 9, 12). En l'absence de portion évidée 16 définie comme ci-avant, la couche de substance épilatoire 5 risquerait d'être laminée entre lesdits premier et deuxième rouleaux 9, 12. Dans le pire des cas, la bande épilatoire 2 serait alors écrasée de façon à faire sortir, par les côtés par exemple, la substance épilatoire ainsi laminée. Ceci pourrait non seulement réduire de manière importante l'efficacité même de la bande épilatoire 2 puisque cette dernière aurait perdu un peu de substance épilatoire et/ou aurait vu l'épaisseur de sa couche de substance épilatoire 5 diminuer, mais également s'avérer salissant pour l'utilisateur, qui reçoit de la substance épilatoire, chaude de surcroît, sur les doigts en saisissant la bande épilatoire 2. Il existe en outre un risque de salissure, voire de mise hors service, d'un appareil de chauffage tel que décrit ci-avant mais dépourvu de la portion évidée 16 telle que définie ci-avant, en raison de l'accumulation de substance épilatoire dans l'appareil.

En d'autres termes, de manière avantageuse, l'appareil de chauffage 1 est conçu de façon qu'il existe :
- un premier écart ou une première zone de contact 17 entre ledit premier rouleau 9 (et plus précisément sa portion d'entrainement 14) et ledit organe de maintien 12, ledit premier écart ou une première zone de contact 17 étant prévu(e) pour accueillir la première zone d'entrainement 13 de la bande épilatoire 2, et
- un second écart S entre ledit premier rouleau 9 (et plus précisément une partie qui n'est pas sa première portion d'entrainement 14) et ledit organe de maintien 12, ledit second écart S délimitant ainsi ladite portion évidée 16 et étant prévu pour accueillir la bande épilatoire 2 au niveau de la couche de substance épilatoire 5. Par exemple, dans le cas où ladite portion évidée 16 fait partie uniquement du premier rouleau 9, comme dans le mode de réalisation illustré aux figures, le premier rouleau 9 comprend ladite première portion d'entrainement 14 et ladite portion évidée 16, chacune formant une fraction de la longueur du premier rouleau 9.

Préférentiellement, lesdits premier écart ou première zone de contact 17 et second écart S se définissent chacun selon un plan respectif perpendiculaire audit premier axe de rotation R₁, chaque plan respectif étant de préférence parallèle au plan A. Le second écart S est donc avantageusement supérieur au premier écart 17 (et bien évidemment supérieur à la première zone de contact 17 qui peut être assimilée à un écart nul ou quasi-nul). En effet, de façon préférentielle, ladite bande épilatoire 2 comprend, au niveau de la couche de substance épilatoire 5, une surépaisseur qu'on ne trouve pas au niveau de la première zone d'entrainement 13, comme l'illustre la figure 13. Il faut donc seulement que ledit premier écart ou ladite première zone de contact 17 permette de laisser passer la première zone d'entrainement 13, qui peut être pincée, coincée et/ou écrasée (entre la première portion d'entrainement 14 et l'organe de maintien 12) puisqu'elle est préférentiellement dépourvue de substance épilatoire, tandis que ledit second écart S permet de laisser passer la couche de substance épilatoire 5, et plus précisément la partie de bande épilatoire 2 comprenant la couche de substance épilatoire 5, sans l'écraser, la pincer, la laminer et/ou la coincer. Autrement dit, ledit second écart S est suffisamment grand pour que ladite portion évidée 16 puisse accueillir ladite couche de substance épilatoire 5 sans que cette dernière ne soit laminée et/ou écrasée entre ledit premier rouleau d'entrainement 9 et ledit organe de maintien 12. De façon avantageuse, ledit second écart S est donc au moins égal à la somme de l'épaisseur de ladite couche de substance dépilatoire 5 et de l'épaisseur du premier substrat 4, et de manière encore plus avantageuse, cette somme inclut également l'épaisseur de la feuille protectrice 6.

Selon l'invention, ledit appareil de chauffage 1 comprend en outre un moyen de chauffage 18 prévu pour chauffer ladite portion évidée 16 de façon à porter ladite substance épilatoire à une température d'utilisation lorsque ladite couche 5 est reçue dans ladite portion évidée 16. En d'autres termes, ledit moyen de chauffage 18 est avantageusement conçu de sorte à chauffer suffisamment l'évidement formé par ladite portion évidée 16 de façon que, lorsque la bande épilatoire 2 est entrainée par le premier rouleau 9, la couche de substance épilatoire 5 se trouvant au sein de la portion évidée 16, et plus précisément de l'évidement formé par cette dernière, est réchauffée à ladite température d'utilisation.

Préférentiellement, ladite température d'utilisation désigne de manière générale une température à laquelle l'efficacité de la bande épilatoire 2 est augmentée par rapport à une température standard (par exemple 20°C), et en particulier la température d'utilisation est comprise entre 25 et 60°C, plus préférentiellement entre 30 et 50 °C, par exemple environ 40°C (+/-3°C). La température d'utilisation peut donc correspondre à toute température à partir de laquelle l'efficacité de la bande épilatoire se trouve améliorée, et toute température au-delà. Par exemple, la température d'utilisation correspond à une température de début de changement de phase (solide vers liquide) de la substance épilatoire, ou une température supérieure. Ainsi, l'appareil de chauffage 1 peut être conçu pour porter la couche de substance épilatoire 5 de la bande épilatoire 2 à une température à laquelle la substance épilatoire, notamment de la cire, est au moins en partie fondue et/ou ramollie.

Le concept de l'invention est donc d'entrainer la bande épilatoire 2 de l'entrée d'alimentation 10 à la sortie 11 de l'appareil 1 en agissant sur une zone de cette bande 2 de préférence dépourvue de substance épilatoire (en l'occurrence, ladite première zone d'entrainement 13), tout en laissant un évidement (formé par la portion évidée 16) chauffé suffisamment grand pour le passage et le chauffage d'une autre zone de la bande épilatoire 2 pourvue de substance épilatoire. Un autre avantage de la configuration de l'appareil de chauffage 1 de l'invention est son faible encombrement. En effet, le premier rouleau 9 permet de faire défiler la bande épilatoire 2 pour la chauffer et en particulier chauffer sa couche de substance épilatoire 5, et il n'est donc pas nécessaire de prévoir un premier rouleau 9 avec un grand diamètre. Au contraire, le premier rouleau 9 peut présenter une longueur sensiblement égale ou légèrement supérieure à la largeur de la bande épilatoire 2 (dans le cas d'une bande épilatoire 2 rectangulaire par exemple), et un diamètre bien inférieur à la longueur de la bande épilatoire 2, par exemple moins d'un quart de celle-ci. Il est important de noter que dans le mode de réalisation préférentiel illustré aux figures, l'appareil de chauffage 1 n'est pas conçu pour que la bande épilatoire s'enroule autour dudit premier rouleau 9, ou même autour dudit deuxième rouleau 12, bien que ces possibilités ne soient pas totalement exclues et pourraient se retrouver dans des modes de réalisation alternatifs de l'invention.

Dans le mode de réalisation particulier illustré aux figures, ledit moyen de chauffage 18 comprend un premier élément de chauffage 19, par exemple une première résistance, ledit premier élément de chauffage 19 étant disposé en regard dudit premier rouleau 9, et notamment le long de ce dernier. Toujours dans ce mode de réalisation particulier, ledit moyen de chauffage 18 comprend en outre un deuxième élément de chauffage 20, par exemple une deuxième résistance, ledit deuxième élément de chauffage 20 étant disposé en regard dudit organe de maintien 12 (en en particulier en regard dudit deuxième rouleau 12). Ainsi, de manière avantageuse, ledit premier rouleau 9 et ledit organe de maintien 12 sont positionnés entre lesdits premier et deuxième éléments de chauffage 19, 20. Le premier élément de chauffage 19 est par exemple conçu pour chauffer le premier rouleau 9, et en particulier chauffer la portion évidée 16 (incluant l'évidement formé par cette dernière) tandis que le deuxième élément de chauffage 20 chauffe l'organe de maintien 12, et plus précisément ledit deuxième rouleau 12, l'organe de maintien 12 et le premier rouleau 9 chauffant ensuite par contact ladite bande épilatoire 2 en particulier au niveau de ladite couche de substance épilatoire 5. Bien évidemment, chaque élément de chauffage 19, 20 peut également permettre de chauffer la portion évidée 16, et plus précisément l'évidement formée par cette dernière et conçu pour recevoir la couche de substance épilatoire 5, par convection. Chaque élément de chauffage 19, 20 peut en outre présenter, comme illustré aux figures, une forme de plaque respective sensiblement rectangulaire et allongée positionnée le long du rouleau correspondant (c'est-à-dire ledit premier rouleau 9 pour une des deux plaques et ledit deuxième rouleau 12 pour une autre des deux plaques). Alternativement, ledit premier élément de chauffage 19 est intégré au sein dudit premier rouleau 9 ou forme au moins en partie ce dernier.

Toujours dans le mode de réalisation illustré aux figures, ledit premier rouleau 9 comprend une deuxième portion d'entrainement 21. De façon avantageuse, ledit organe de maintien 12 est alors conçu pour maintenir en contact au moins une deuxième zone d'entrainement 22 de ladite bande épilatoire 2 avec ladite deuxième portion d'entrainement 21, afin de faciliter l'entrainement de ladite bande épilatoire 2 par ledit premier rouleau 9, et aussi, avantageusement, par ledit deuxième rouleau 12. Une telle configuration permet un entrainement particulièrement réussi et homogène de la bande épilatoire 2 par l'appareil 1 et en particulier par le premier rouleau 9.

Plus avantageusement encore, le long dudit premier rouleau 9, lesdites première et deuxième portions d'entrainement 14, 21 sont positionnées de part et d'autre de ladite portion évidée 16. Celle-ci est donc, selon cette caractéristique préférentielle, positionnée de manière centrale relativement auxdites première et deuxième portions d'entrainement 14, 21. Bien évidemment, lesdites première et deuxième portions d'entrainement 14, 21 sont de préférence distinctes, et situées à distance l'une de l'autre. De même, lesdites première et deuxième zones d'entrainement 13, 22 de ladite bande épilatoire 2 sont avantageusement distinctes, et situées à distance l'une de l'autre, comme illustré aux figures 12 à 14. Ladite deuxième zone d'entrainement 22 est par exemple formée par un second bord de ladite bande épilatoire 2, ledit second bord étant positionné à l'opposé du premier bord. De préférence, ladite deuxième zone d'entrainement 22 est sensiblement dépourvue de substance épilatoire, et correspond par exemple à une zone où sont collées directement l'un à l'autre un bord du premier substrat 4 et un bord de la feuille protectrice 6.

Selon le mode de réalisation illustré aux figures, ladite première portion d'entrainement 14 est formée par un épaulement. De préférence, ladite deuxième portion d'entrainement 21 est également formée par un épaulement, distinct du précédent. Ainsi, ledit premier rouleau 9 présente ici une forme générale d'haltère, avec une barre centrale comprenant la portion évidée 16 en son centre et deux anneaux (par analogie à des poids d'un haltère ayant une forme spécifique) enfilés de part et d'autre sur cette barre et formant lesdites première et deuxième portions d'entrainement 14, 21 sous la forme de deux épaulements. Optionnellement, ladite première portion d'entrainement 14 est réglable de façon à pouvoir être déplacée le long dudit premier rouleau 9, par exemple par vissage ou pincement, permettant ainsi de modifier une dimension de ladite portion évidée 16, par exemple sa longueur. Selon une autre option de réalisation, ladite deuxième portion d'entrainement 21 est réglable de façon à pouvoir être déplacée le long dudit premier rouleau 9, par exemple par vissage ou pincement, permettant ainsi de modifier une dimension de ladite portion évidée 16, par exemple sa longueur. Chaque portion d'entrainement 14, 21 peut ainsi être optionnellement formée par un anneau respectif, par exemple fixé le long d'un cylindre constituant le corps central du premier rouleau 9. Bien évidemment, les mêmes options peuvent également exister pour le deuxième rouleau 12 (indépendamment du premier rouleau 9), surtout lorsque ladite partie évidée 16 est prévue au moins en partie dans ledit deuxième rouleau 12. Dans ce dernier cas (non illustré), ledit deuxième rouleau 12 présente des troisième et quatrième portions d'entrainement aux fonctions similaires respectivement à celles desdites première et deuxième portions d'entrainement 14, 21.

Selon un mode de réalisation particulier de l'appareil de chauffage 1, compatible avec celui représenté aux figures, ledit premier rouleau 9 et/ou ledit organe de maintien est/sont 12 amovible(s), de façon à pouvoir modifier au moins une dimension de ladite portion évidée 16, par exemple sa longueur et/ou sa profondeur, par remplacement dudit premier rouleau 9 et/ou dudit organe de maintien 12 par un autre présentant au moins une dimension différente. En d'autres termes, l'appareil de chauffage 1 est conçu de manière à ce que ledit premier rouleau 9 et/ou ledit organe de maintien 12 peu(ven)t être aisément démonté(s) du reste de l'appareil 1, et remplacé(s) par un élément similaire mais présentant au moins une dimension différente, afin de faire varier la taille de la portion évidée 16, et notamment sa longueur et/ou sa profondeur.

Alternativement, ladite première portion d'entrainement 14 est formée par une roue dentée (non illustrée), en lieu et place de l'épaulement mentionné précédemment ou encore en combinaison avec ce dernier. Ladite première zone d'entrainement 13 de ladite bande épilatoire 2 comprend alors avantageusement une pluralité de trous traversants alignés, les dents de la roue dentée étant destinées à s'insérer dans lesdits trous traversants pour entrainer ladite bande épilatoire 2 lors de la rotation dudit premier rouleau 9. La configuration de ce mode de réalisation alternatif permet de minimiser le risque d'erreur de positionnement de la bande épilatoire 2 entre le premier rouleau 9 et l'organe de maintien 12 en contrôlant l'insertion de cette dernière au sein de ladite portion évidée 16. En revanche, cette configuration demande une étape de réalisation des trous traversants dans la première zone d'entrainement 13, de préférence formée par un bord latéral, de la bande épilatoire 13, et un placement précis de la bande afin de faire correspondre les trous traversants avec les dents de la roue dentée. Optionnellement, ladite deuxième portion d'entrainement 21 est elle aussi formée par une roue dentée, distincte de la précédente mais aux fonctions similaires. Dans ce dernier cas, la deuxième zone d'entrainement 22 de la bande épilatoire 2 comprend elle aussi une pluralité de trous traversants alignés, distincts des précédents mais aux fonctions similaires.

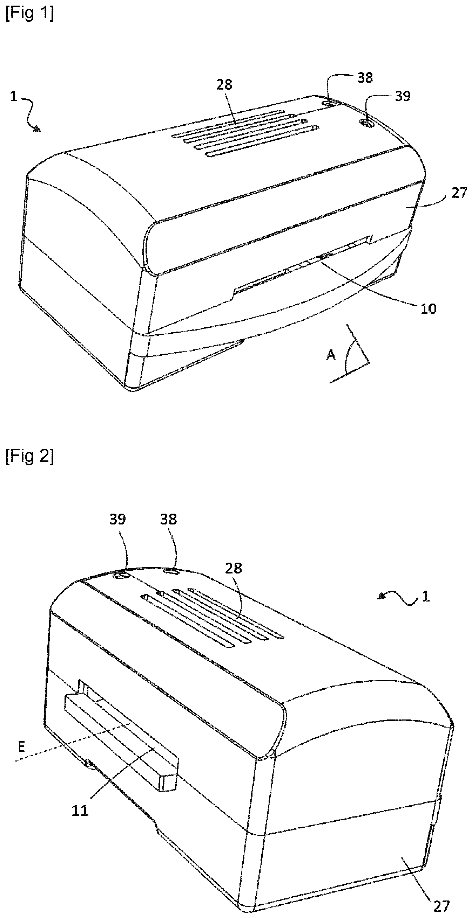

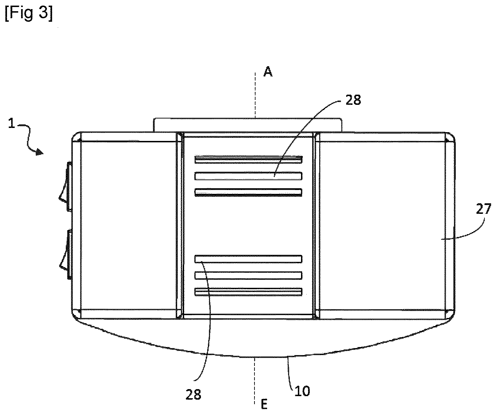

Selon un mode de réalisation illustré aux figures, et en particulier aux figures 1 à 5 et 8, l'appareil de chauffage 1 comprend en outre, une enceinte (ou carter) 27 contenant la plupart, voire la totalité, des autres composants, et en particulier au moins ledit premier rouleau 9, ledit organe de maintien 12, et ledit moyen de chauffage 18. Une telle configuration notamment de mieux contrôler la chaleur à l'intérieur de l'enceinte 27, et donc la température à laquelle va être portée ladite bande épilatoire 2 grâce à l'appareil de chauffage 1. Préférentiellement, ladite enceinte 27 est pourvue, outre une ouverture pour l'entrée d'alimentation 10 et une ouverture pour la sortie d'enlèvement 11 de la bande épilatoire 2, d'ouvertures 28 permettant un refroidissement contrôlé de l'intérieur de l'enceinte 27. Ladite enceinte 27 peut être formée de deux demi-coques mises en correspondance l'une avec l'autre.

Avantageusement, l'appareil de chauffage 1 comprend également au moins un capteur de température (non illustré), permettant de savoir quand la température atteinte par l'actionnement du moyen de chauffage 18 au sein de l'appareil 1 est suffisante pour chauffer ladite bande épilatoire 2, et en particulier pour porter ladite couche de substance épilatoire 5 à une température d'utilisation.

Selon un mode de réalisation particulier de l'invention, et comme illustré aux figures 4, 5 et 8 notamment, l'appareil de chauffage 1 comprend en outre, au niveau de ladite sortie d'enlèvement 11, un système de réception de ladite bande épilatoire 2 chauffée, comprenant par exemple un plateau de réception 29. Ledit système de réception peut également comprendre un système de guidage, en sortie de l'appareil de chauffage 1, de ladite bande épilatoire 2, ledit système de guidage pouvant par exemple être au moins en partie formé par ledit plateau de réception 29. Une telle configuration permet notamment de disposer d'un stock de bandes épilatoires 2 chauffées par l'appareil 1 dans un endroit déterminé. Plus préférentiellement, ledit système de réception est rétractable. En particulier, ledit plateau de réception 29 et/ou ledit système de guidage peut être escamotable à l'intérieur de ladite enceinte 27 ou dans le corps même de cette dernière. Une telle configuration permet notamment de diminuer l'encombrement de l'appareil de chauffage 1 lorsqu'il n'est pas utilisé.

Selon le mode de réalisation illustré aux figures, ledit système de réception comprend en outre un moyen de conservation de la chaleur, par exemple une enveloppe secondaire 30 disposée autour du plateau de réception 29, afin de garder le plus longtemps possible ladite bande épilatoire 2 à température d'utilisation. Dans le mode de réalisation illustré aux figures, l'enveloppe secondaire 30 fait partie d'une platine secondaire 32 enveloppant en partie au moins ledit deuxième élément de chauffage 20 et ledit deuxième rouleau 12, et se prolongeant au-dessus du plateau de réception 29. Bien entendu, une portion de ladite enceinte 27 peut également former une partie de l'enveloppe secondaire 30.

Selon encore un autre mode de réalisation particulier (non illustré) de l'invention, compatible avec les précédents, ledit système de réception comprend un élément de chauffage de sortie, tel qu'une troisième résistance, ledit élément de chauffage de sortie étant conçu pour maintenir à température d'utilisation ladite bande épilatoire 2, en particulier après qu'elle ait été chauffée par l'appareil de chauffage 1. Une telle configuration pourrait par exemple s'illustrer par un positionnement dudit élément de chauffage de sortie sous le plateau de réception 29, au niveau ou juste avant la sortie d'enlèvement 11. Dans le mode de réalisation illustré aux figures, le plateau de réception 29 fait partie d'une platine primaire 31 enveloppant en partie au moins ledit premier élément de chauffage 18 et ledit premier rouleau 9, et se prolongeant au-dessous de l'enveloppe secondaire 30. Bien entendu, l'appareil de chauffage 1 comprend avantageusement ladite platine primaire 31 et/ou ladite platine secondaire 32.

Dans le mode de réalisation particulier illustré aux figures, l'appareil de chauffage 1 comprend un premier et un second élément de structure 33, 34 chacun sous la forme d'une plaque respective pourvue d'orifices et/ou d'alésage et/ou de paliers pour guider et/ou supporter plusieurs des éléments mécaniques de l'appareil 1 tels que les platines primaire 31 et secondaire 32, le premier rouleau d'entrainement 9, l'organe de maintien 12, le moteur d'entrainement 23, etc. Chaque élément de structure 33, 34 est de préférence fixe par rapport au premier rouleau 9 (et au deuxième rouleau 12), et fait ainsi partie du bâti de l'appareil de chauffage 1. Les premier et second éléments de structure 33, 34 sont avantageusement positionnés de part et d'autre du premier et du deuxième rouleau 9, 12, sensiblement perpendiculairement à leurs premier et deuxième axes de rotation R₁, R₂. L'appareil de chauffage 1 comprend par exemple un premier orifice traversant 35, lequel est avantageusement pratiqué dans ledit premier élément de structure 33, ledit premier orifice traversant 35 étant destiné à recevoir une portion (notamment une première extrémité) dudit premier et/ou dudit deuxième rouleau 9, 12 (dans le cas illustré aux figures, c'est le deuxième rouleau 12 qui est reçu par le premier orifice 35), ledit premier et/ou deuxième rouleau 9, 12 étant préférentiellement monté à rotation par rapport auxdits éléments de structure 33, 34.

De manière particulièrement avantageuse, l'appareil de chauffage 1 est conçu de manière à ce que lesdits premier rouleau 9 et organe de maintien 12 (et plus précisément lesdits premier et deuxième rouleaux 9, 12) puissent s'écarter davantage l'un de l'autre sans rompre ladite chaîne cinématique, lesdits premier et deuxième pignons d'entrainement 24, 25, restant associés l'un à l'autre de manière à former un engrenage. Cette configuration est particulièrement intéressante dans le cas où un élément d'insertion un peu trop gros est inséré dans ledit appareil de chauffage 1, entre lesdits premier rouleau 9 et organe de maintien 12 (et plus précisément entre lesdits premier et deuxième rouleaux 9, 12), ledit élément d'insertion étant par exemple formé d'une ou plusieurs bandes épilatoires 2, d'une bande épilatoire pliée, etc...). Il est ainsi possible de continuer le mouvement de rotation du premier rouleau 9 au moins lorsque l'élément d'insertion n'est pas trop gros, évitant ainsi à la fois un « *bourrage* » (et donc un arrêt non désiré) de l'appareil de chauffage 1 et une dégradation par écrasement dudit élément d'insertion qui peut en particulier être formé d'une bande épilatoire 2 ou d'une pluralité de bandes épilatoires 2. Par exemple, ledit premier orifice traversant 35 présente une forme permettant un débattement de la portion du (premier ou deuxième) rouleau 9 ou 12 inséré dedans (ici, c'est une portion du deuxième rouleau 12 qui est inséré dedans). En d'autres termes, le premier orifice 35 est de préférence sensiblement plus grand qu'une section transversale de la portion du rouleau 9 ou 12 insérée dans ledit premier orifice 35. Ce débattement permet un écartement supplémentaire entre le premier rouleau 9 et l'organe de maintien 12 (plus précisément dans l'exemple illustré, le deuxième rouleau 12) lorsqu'un élément légèrement trop gros pour passer par l'évidement de la portion évidée 16 est inséré dans l'appareil de chauffage 1. En s'écartant du premier rouleau 9, le deuxième pignon 25 du deuxième rouleau 12 reste accouplé audit premier pignon 24 et continue d'être mis en mouvement par ledit moteur d'entrainement 23, puisque dans l'exemple illustré aux dessins, le moteur 23 est couplé au premier pignon 24 via l'élément rotatif récepteur 26, le premier pignon 24 étant couplé au deuxième pignon 25 pour l'entrainer en rotation. Il suffit pour cela par exemple que les premier et deuxième pignons d'entrainement 24, 25 forment entre eux des engrenages plus profonds que l'écartement supplémentaire entre lesdits premier rouleau 9 et organe de maintien 12. De manière avantageuse, l'appareil de chauffage 1 comprend un second orifice traversant (non visible aux figures), de préférence pratiqué dans ledit second élément de structure 34, et destiné à recevoir une portion (notamment une seconde extrémité, opposée à ladite première extrémité) dudit premier et/ou dudit deuxième rouleau 9, 12 (dans l'exemple des figures 6 et 7, le deuxième rouleau 12 est concerné), ce second orifice traversant présentant les mêmes fonction et structure que le premier orifice 35, c'est-à-dire, un débattement permettant l'écartement du premier rouleau 9 et de l'organe de maintien 12. Chaque débattement peut se traduire par exemple comme une forme oblongue dudit premier et/ou dudit second orifice 35 tandis que la partie dudit premier et/ou deuxième rouleau 9, 12 insérée dans l'orifice présente par exemple une forme localement cylindrique à base circulaire. L'appareil de chauffage 1 comprend de préférence un premier élément de rappel 37, tel qu'une tige élastique (et donc flexible) de rappel, ledit premier élément de rappel 37 étant agencé de manière à rappeler ledit premier rouleau 9 et/ou ledit organe de maintien 12 (plus précisément ledit deuxième rouleau 12) à sa position initiale après un écartement tel que décrit ci-avant. Ledit premier élément de rappel 37 est de préférence seulement en appui sur ledit premier rouleau 9 et/ou ledit deuxième rouleau 12 et non lié au rouleau, de manière à faciliter le remplacement du rouleau 9, 12 ou du premier élément de rappel 37. Dans l'exemple illustré aux figures 6, 7 et 10, la tige élastique de rappel est disposée perpendiculairement, en regard dudit premier orifice 35 audit deuxième rouleau 12 de manière à exercer un effort sur une première extrémité de ce rouleau en direction du premier rouleau 9. Une autre tige élastique de rappel semblable peut être prévue en regard dudit second orifice.

Néanmoins, il est tout à fait possible qu'il se produise tout de même un bourrage total de l'appareil de chauffage 1, c'est-à-dire un blocage de ce dernier lorsqu'un objet de trop grande taille (et notamment plus gros que l'élément d'insertion) est inséré dans ledit appareil de chauffage 1, entre lesdits premier rouleau 9 et organe de maintien 12 (et plus précisément entre lesdits premier et deuxième rouleaux 9, 12). Ledit objet de trop grande peut par exemple être un objet non prévu pour être inséré dans l'appareil de chauffage 1 (doigt humain, stylo, vêtement, etc.). Ce blocage est généralement appelé « *bourrage* » et désigne notamment un blocage mécanique du moteur d'entraînement 23. Ainsi, de façon avantageuse, comme illustré aux figures 6 et 7, l'appareil de chauffage 1 comprend en outre un système de débrayage pour désaccoupler ledit moteur d'entrainement 23 dudit premier rouleau 9 lorsqu'un objet de trop grande taille est inséré entre ledit premier rouleau 9 et ledit organe de maintien 12 (et plus précisément entre lesdits premier et deuxième rouleaux 9, 12). Ce système de débrayage est en particulier conçu pour stopper l'entraînement par ledit moteur d'entrainement 23 dudit premier rouleau 9, ce qui provoque préférentiellement également l'arrêt de l'entraînement (indirect, puisque via le premier rouleau 9) dudit deuxième rouleau 12 par ledit moteur d'entrainement 23.

Par exemple, le système de débrayage comprend un élément de débrayage, permettant à un utilisateur de pousser manuellement, de préférence depuis l'extérieur de l'appareil de chauffage 1, ledit moteur d'entrainement 23. Ledit élément de débrayage comprend de préférence un bouton de débrayage (non illustré) affleurant ou faisant saillie de ladite enceinte 27. Ledit élément de débrayage comprend en outre avantageusement une platine de débrayage 40 bordant une partie au moins dudit moteur d'entrainement 23. Ledit bouton de débrayage est ainsi préférentiellement relié à ladite platine de débrayage 40, de sorte que lorsqu'un utilisateur appuie sur ledit bouton de débrayage, ou plus généralement lorsqu'une force de pression Pₑ est exercée sur un côté prédéterminé de ladite platine de débrayage 40, cette dernière se déplace selon une direction de déplacement D_{d} pour pousser ledit moteur d'entrainement 23. Ledit système de débrayage est alors conçu pour que, sous la force de pression exercée Pₑ, transmise audit moteur d'entrainement 23, ce dernier puisse pivoter autour d'un axe de pivotement P de façon à se désaccoupler du premier rouleau 9, rompant ainsi avantageusement, de manière réversible et temporaire, ladite chaîne cinématique. A la figure 6, le pivotement entraînant un désaccouplement du moteur 23 et du premier rouleau 9 est, d'un point de vue situé à gauche de la vue de cette figure, réalisé selon un sens horaire autour de l'axe de pivotement P. Le désaccouplement dudit premier rouleau 9 et dudit moteur d'entrainement 23 est de préférence réalisé, lorsque le système de débrayage est actionné et plus précisément lorsque ladite platine de débrayage 40 est poussée), grâce à un écartement relatif du premier pignon 24 et dudit l'élément rotatif récepteur 26, qui ne sont alors plus accouplés. L'utilisateur va alors pouvoir tirer l'objet ayant bourré le système puisque le ou les (premier et/ou deuxième) rouleau(x) 9, 12 ne sont plus couplés au moteur et ne vont donc opposer aucune résistance. Le système de débrayage susvisé forme donc un système de sécurité qui permet d'éviter non seulement des accidents de personne (un utilisateur se coinçant le doigt dans l'appareil 1 par exemple), mais également de diminuer les risques de dégradation de l'appareil 1 suite à une mauvaise manipulation de ce dernier telle que l'insertion d'un doigt, de plusieurs bandes épilatoires 2 en même temps, d'une bande épilatoire pliée plusieurs fois, d'un crayon, et tout autre erreur d'utilisation entrainant un « *bourrage* » de l'appareil de chauffage 1. Le système de débrayage susvisé forme en particulier un système permettant la remise en marche rapide de l'appareil de chauffage 1 en cas d'arrêt intempestif de ce dernier par blocage mécanique.

De manière avantageuse, comme illustré aux figures 6 et 7, ledit système de débrayage comprend un second élément de rappel 36 conçu pour remettre ledit moteur d'entrainement 23 en position d'accouplement avec ledit premier rouleau. A la figure 6, ce retour en position d'accouplement s'effectue également par un pivotement autour de l'axe de pivotement P, cette fois réalisé selon un sens antihoraire. Ledit élément de rappel 36 est préférentiellement relié au moteur d'entrainement 23 et est par exemple formé par un ressort de rappel 36. Dans le mode de réalisation des figures 6 et 7, ledit moteur d'entrainement 23 est relié à pivotement audit premier élément de structure 33 selon une course angulaire prédéterminée et limitée, de préférence inférieure à 10°, plus préférentiellement inférieure à 5°, tandis que ledit ressort de rappel 36 est relié audit premier élément de structure 33. Le moteur 23 est donc avantageusement monté à rotation avec le bâti de l'appareil de chauffage 1.

En résumé, ledit système de débrayage est donc avantageusement conçu pour faire passer le premier rouleau 9 (et ledit deuxième rouleau 12) :
- d'une position de débrayage, lorsqu'un objet de trop grande taille est inséré entre lesdits premier rouleau 9 et organe de maintien 12 (par exemple dans la portion évidée 16), le premier pignon 24 et l'élément rotatif récepteur 26 s'écartant l'un de l'autre de façon que ledit premier rouleau 9 soit désaccouplé du moteur 23, ledit premier rouleau 9 donc plus mis en mouvement par ce dernier;
- à une position de couplage (qui est de préférence une position de rappel), lorsqu'aucun objet de trop grande taille n'est inséré entre lesdits premier rouleau 9 et organe de maintien 12, ledit rouleau 9 étant alors accouplé audit moteur d'entrainement 23, l'élément rotatif récepteur 26 et ledit premier pignon 24 étant rappelés en accouplement en particulier grâce audit ressort de rappel 36,
   et inversement.

Selon un mode de réalisation particulier, l'appareil de chauffage 1 est conçu pour fonctionner sur secteur, grâce par exemple à une prise électrique d'un bâtiment, telle qu'une prise électrique de salle de bain, de salon, etc. Selon un autre mode de réalisation particulier, l'appareil de chauffage 1 est conçu pour fonctionner sur batterie. Cette dernière configuration est particulièrement avantageuse pour l'utilisation nomade, c'est-à-dire en déplacement, de l'appareil de chauffage 1. Il est possible, grâce à ce mode de réalisation de l'invention, de chauffer facilement et rapidement des bandes épilatoires 2 pour augmenter de manière significative leur efficacité et leur rapidité d'application, et ce même lorsque l'utilisateur n'est pas dans des conditions idéales pour s'épiler, et notamment en vacances, en déplacement professionnel ou familial, etc. L'appareil de chauffage 1 peut éventuellement inclure un chargeur dédié pour charger la batterie, ledit chargeur pouvant selon un exemple particulier être un chargeur classique de téléphone portable, d'ordinateur portable, d'un autre appareil lié à l'hygiène corporelle, etc. Bien évidemment, il est aussi envisageable que l'appareil de chauffage 1 puisse fonctionner alternativement ou en combinaison sur secteur et sur batterie, par exemple à la manière d'un ordinateur portable pourvu d'une batterie se chargeant sur secteur.

L'invention concerne, selon un troisième aspect, un procédé de chauffage d'au moins une bande épilatoire 2, laquelle est de préférence celle mentionnée ci-avant, le procédé étant de préférence mis en oeuvre à l'aide de l'appareil de chauffage décrit ci-avant. Ainsi, ladite bande épilatoire 2 comprend au moins un premier substrat 4 et une couche de substance épilatoire 5, par exemple une cire, préenduite sur ledit premier substrat 4. La description qui précède, comme celle qui suit, concernant l'appareil de chauffage 1 et le kit d'épilation s'applique donc également au procédé de chauffage selon l'invention, et inversement, la description qui suit concernant le procédé de chauffage s'applique avantageusement également à l'appareil de chauffage 1 et au kit d'épilation selon l'invention.

Selon l'invention, le procédé de chauffage comprend au moins :
- une étape de mise en contact de ladite bande épilatoire 2 avec un premier rouleau d'entrainement 9 et un organe de maintien 12, l'organe de maintien 12 permettant de maintenir en contact au moins une première zone d'entrainement 13 de ladite bande épilatoire 2 avec au moins une première portion d'entrainement 14 dudit premier rouleau d'entrainement 9,
- une étape de mise en rotation dudit premier rouleau d'entrainement 9 de façon à entraîner ladite bande épilatoire 2 d'une entrée d'alimentation 10 vers une sortie d'enlèvement 11 de l'appareil de chauffage 1,
- une étape d'insertion de ladite couche 5 dans une portion évidée 16 du premier rouleau d'entrainement 9 et/ou de l'organe de maintien 12, et
- une étape de chauffage de ladite portion évidée 16 de façon à porter ladite substance épilatoire à une température d'utilisation lorsque ladite couche 5 est insérée dans ladite portion évidée 16.

De préférence, ladite étape d'insertion est au moins partiellement concomitante à l'étape de mise en rotation, celles-ci étant au moins partiellement concomitantes avec ladite étape de chauffage. Naturellement, ladite étape de chauffage est préférentiellement réalisée à l'aide dudit moyen de chauffage 18.

Avantageusement, le procédé comprend en outre une étape d'écrasement et/ou de pincement de ladite première zone d'entrainement 13, lors de laquelle cette dernière est écrasée et/ou pincée au moins :
- par ledit premier rouleau d'entrainement 9, et plus précisément la première portion d'entrainement 14 de ce dernier,
- et de préférence également par ledit organe de maintien 12, et notamment ledit deuxième rouleau 12.

Bien évidemment, ladite étape d'insertion correspond préférentiellement à la réception par ladite portion évidée 16 de ladite couche de substance épilatoire 5, et plus globalement de la partie de bande épilatoire 2 comprenant ladite couche 5 (à l'exclusion donc des première et deuxième zones d'entrainement 13, 22).

De manière avantageuse, l'appareil de chauffage 1 comprend au moins un premier et un second témoin lumineux 38, 39, visibles aux figures 1, 2, 4 et 5 notamment. Ladite étape de chauffage comprend de préférence :
- une étape d'atteinte d'une température de consigne, par exemple au sein de l'appareil de chauffage 1 et détectée grâce audit capteur de température,
- une première étape d'avertissement lumineux, au cours de laquelle une première couleur (par exemple rouge) est émise, par exemple par ledit premier témoin lumineux 38, pour avertir l'utilisateur que la température de consigne n'est pas atteinte,
- une seconde étape d'avertissement lumineux, au cours de laquelle une seconde couleur (par exemple verte), différente de la première couleur, est émise, par exemple par ledit second témoin lumineux 39, pour avertir l'utilisateur que la température de consigne est atteinte, et donc que de manière avantageuse, l'appareil de chauffage 1 est prêt à être utilisé pour réchauffer la bande épilatoire 2.

Avantageusement, lors de la première étape d'avertissement lumineux, le second témoin lumineux 39 est éteint et le premier témoin lumineux 38 est allumé, et lors de la seconde étape d'avertissement lumineux, le premier témoin lumineux 38 est éteint et le second témoin lumineux 39 est allumé.

La température de consigne est de préférence supérieure ou égale à la température d'utilisation.

Dans les différents modes de réalisation et exemples présentés précédemment, toutes les combinaisons techniquement possibles des caractéristiques présentées peuvent faire partie de l'objet de l'invention.

L'invention permet ainsi de chauffer de façon extrêmement simple, efficace et pratique une bande épilatoire, et ce dans des conditions de sécurité optimales.

## Revendications

1. Appareil de chauffage (1) d'au moins une bande épilatoire (2), ladite bande épilatoire (2) comprenant au moins un premier substrat (4) et une couche de substance épilatoire (5), par exemple une cire, préenduite sur ledit premier substrat (4), ledit appareil de chauffage (1) étant **caractérisé en ce qu'**il comprend au moins :
- un premier rouleau d'entrainement (9) conçu pour être mis en rotation de façon à déplacer ladite bande épilatoire (2) d'une entrée d'alimentation (10) vers une sortie d'enlèvement (11) de l'appareil de chauffage (1), et
- un organe de maintien (12) pour maintenir en contact au moins une première zone d'entrainement (13) de ladite bande épilatoire (2) avec au moins une première portion d'entrainement (14) dudit premier rouleau d'entrainement (9), afin de permettre l'entrainement de ladite bande épilatoire (2) par ledit premier rouleau (9),
ledit premier rouleau d'entrainement (9) et/ou ledit organe de maintien (12) comprenant une portion évidée (16) destinée à accueillir ladite couche (5) lorsque ladite bande épilatoire (2) est entrainée par ledit premier rouleau d'entrainement (9), ledit appareil de chauffage (1) comprenant en outre un moyen de chauffage (18) prévu pour chauffer ladite portion évidée (16) de façon à porter ladite substance épilatoire à une température d'utilisation lorsque ladite couche (5) est reçue dans ladite portion évidée (16),
ledit organe de maintien (12) étant formé par un deuxième rouleau d'entrainement (12), et **en ce que** ledit appareil de chauffage (1) comprend en outre un système d'entrainement (15) desdits premier et deuxième rouleaux (9, 12) en mouvement contrarotatif l'un par rapport à l'autre pour entraîner le déplacement de ladite bande épilatoire (2).

2. Appareil de chauffage (1) selon la revendication précédente, **caractérisé en ce que** les premier et deuxième rouleaux (9, 12) sont agencés en regard l'un de l'autre et le long l'un de l'autre

3. Appareil de chauffage (1) selon la revendication 1 ou 2, **caractérisé en ce que** ledit moyen de chauffage (18) comprend un premier élément de chauffage (19), par exemple une première résistance, ledit premier élément de chauffage (19) étant disposé en regard dudit premier rouleau (9).

4. Appareil de chauffage (1) selon l'une quelconque des revendications précédentes, **caractérisé en ce que** ledit moyen de chauffage (18) comprend en outre un deuxième élément de chauffage (20), par exemple une deuxième résistance, ledit deuxième élément de chauffage (20) étant disposé en regard dudit organe de maintien (12).

5. Appareil de chauffage (1) selon les revendications 3 et 4, **caractérisé en ce que** ledit premier rouleau (9) et ledit organe de maintien (12) sont positionnés entre lesdits premier et deuxième éléments de chauffage (19, 20).

6. Appareil de chauffage (1) selon l'une quelconque des revendications précédentes, **caractérisé en ce que** ledit premier rouleau (9) comprend une deuxième portion d'entrainement (21), et **en ce que** ledit organe de maintien (12) est conçu pour maintenir en contact au moins une deuxième zone d'entrainement (22) de ladite bande épilatoire (2) avec ladite deuxième portion d'entrainement (21), afin de faciliter l'entrainement de ladite bande épilatoire (2) par ledit premier rouleau (9).

7. Appareil de chauffage (1) selon la revendication précédente, **caractérisé en ce que** le long dudit premier rouleau (9), lesdites première et deuxième portions d'entrainement (14, 21) sont positionnées de part et d'autre de ladite portion évidée (16).

8. Appareil de chauffage (1) selon l'une quelconque des revendications précédentes, caractérisé en ce ladite première portion d'entrainement (14) est réglable de façon à pouvoir être déplacée le long dudit premier rouleau (9), par exemple par vissage ou pincement, permettant ainsi de modifier une dimension de ladite portion évidée (16), par exemple sa longueur.

9. Appareil de chauffage (1) selon l'une quelconque des revendications précédentes, **caractérisé en ce que** ladite première portion d'entrainement (14) est formée par un épaulement.

10. Appareil de chauffage (1) selon l'une quelconque des revendications 1 à 8, **caractérisé en ce que** ladite première portion d'entrainement (14) est formée par une roue dentée, ladite première zone d'entrainement (13) comprenant une pluralité de trous traversants alignés, les dents de la roue dentée étant destinées à s'insérer dans lesdits trous traversants pour entrainer ladite bande épilatoire (2) lors de la rotation dudit premier rouleau (9).

11. Appareil de chauffage (1) selon l'une quelconque des revendications précédentes, **caractérisé en ce que** ledit premier rouleau (9) et/ou ledit organe de maintien (12) est/sont amovible(s), de façon à pouvoir modifier au moins une dimension de ladite portion évidée (16), par exemple sa longueur et/ou sa profondeur, par remplacement dudit premier rouleau (9) et/ou dudit organe de maintien (12) par un autre présentant au moins une dimension différente.

12. Appareil de chauffage (1) selon l'une quelconque des revendications précédentes, **caractérisé en ce qu'**il comprend en outre, au niveau de ladite sortie d'enlèvement (11), un système de réception de ladite bande épilatoire (2) chauffée, comprenant par exemple un plateau de réception (29).

13. Appareil de chauffage (1) selon la revendication précédente, **caractérisé en ce que** ledit système de réception est rétractable.

14. Appareil de chauffage (1) selon la revendication 12 ou 13, **caractérisé en ce que** ledit système de réception comprend un moyen de conservation de la chaleur, par exemple une enveloppe secondaire (30) disposée autour du plateau de réception (29), afin de garder le plus longtemps possible ladite bande épilatoire (2) à température d'utilisation.

15. Appareil de chauffage (1) selon l'une des revendications 12 à 14, **caractérisé en ce que** ledit système de réception comprend un élément de chauffage de sortie, tel qu'une troisième résistance, ledit élément de chauffage de sortie étant conçu pour maintenir à température d'utilisation ladite bande épilatoire (2).

16. Appareil de chauffage (1) selon l'une quelconque des revendications précédentes, **caractérisé en ce qu'**il comprend en outre un moteur d'entrainement (23) pour entrainer en rotation au moins ledit premier rouleau (9), et un système de débrayage pour désaccoupler ledit moteur d'entrainement (23) dudit premier rouleau (9) lorsqu'un objet de trop grande taille est inséré entre ledit premier rouleau (9) et ledit organe de maintien (12).

17. Appareil de chauffage (1) selon l'une quelconque des revendications précédentes, **caractérisé en ce que** ladite première zone d'entrainement (13) de ladite bande épilatoire (2) est sensiblement dépourvue de substance épilatoire.

18. Kit d'épilation comprenant un appareil de chauffage (1) selon l'une quelconque des revendications précédentes, et au moins une bande épilatoire (2) telle que définie dans l'une quelconque des revendications précédentes.

19. Kit d'épilation selon la revendication précédente, **caractérisé en ce qu'**il comprend une pluralité de bandes épilatoires (2) distinctes, de préférence prédécoupées.

20. Procédé de chauffage d'au moins une bande épilatoire (2), ladite bande épilatoire (2) comprenant au moins un premier substrat (4) et une couche de substance épilatoire (5), par exemple une cire, préenduite sur ledit premier substrat (4), le procédé de chauffage étant **caractérisé en ce qu'**il comprend au moins :
- une étape de mise en contact de ladite bande épilatoire (2) avec un premier rouleau d'entrainement (9) et un organe de maintien (12), l'organe de maintien (12) permettant de maintenir en contact au moins une première zone d'entrainement (13) de ladite bande épilatoire (2) avec au moins une première portion d'entrainement (14) dudit premier rouleau d'entrainement (9), ledit organe de maintien (12) étant formé par un deuxième rouleau d'entrainement (12),
- une étape de mise en rotation par un système d'entrainement (15) desdits premier et deuxième rouleaux (9, 12) en mouvement contrarotatif l'un par rapport à l'autre de façon à entraîner ladite bande épilatoire (2) d'une entrée d'alimentation (10) vers une sortie d'enlèvement (11) de l'appareil de chauffage (1),
- une étape d'insertion de ladite couche (5) dans une portion évidée (16) du premier rouleau d'entrainement (9) et/ou de l'organe de maintien (12), et
- une étape de chauffage de ladite portion évidée (16) de façon à porter ladite substance épilatoire à une température d'utilisation lorsque ladite couche (5) est insérée dans ladite portion évidée (16).
